# EUROPÄISCHE PATENTSCHRIFT

(11) **EP 2 856 114 B1**
(45) Veröffentlichungstag und Bekanntmachung des Hinweises auf die Patenterteilung: **14.12.2016**
(21) Anmeldenummer: 13716725.0
(22) Anmeldetag: 15.03.2013
(51) Int. Cl.: G01N 21/33, C02F 1/32, C02F 1/72

(54) **VERFAHREN UND VORRICHTUNG ZUR BESTIMMUNG DES RADIKALZEHRUNGSPOTENTIALS**
METHOD AND DEVICE FOR DETERMINING RADICAL SCAVENGING POTENTIAL
PROCÉDÉ ET DISPOSITIF POUR DÉTERMINER LE POTENTIEL DE CONSOMMATION DE RADICAUX

(30) Priorität: 30.05.2012 DE 102012010611
(43) Veröffentlichungstag der Anmeldung: 08.04.2015
(73) Patentinhaber: Xylem Water Solutions Herford GmbH, 32051 Herford (DE)
(72) Erfinder: RIED, Achim, 32549 Bad Oeynhausen (DE); SCHEIDELER, Jens, 33758 Schloss-Holte (DE); WIELAND, Arne, 32427 Minden (DE)
(74) Vertreter: Lenzing Gerber Stute
(86) Internationale Anmeldenummer: PCT/EP2013/000799
(87) Internationale Veröffentlichungsnummer: WO 2013/178304

(56) Entgegenhaltungen:
- WO-A1-2008/049484
- US-A1- 2005 218 082
- Rosenfeldt E J: "UV Advanced Oxidation Treatment of Emerging Contaminants in Drinking and Reuse Water", , 30. September 2011 (2011-09-30), XP002697635, Gefunden im Internet: URL:http://www.tawwa.org/tw11paper/UV%20Ad vanced%20Oxidation%20Treatment%20of%20Emer ging%20Contaminants%20in%20Drinking%20and% 20Reuse%20Water.pdf [gefunden am 2013-05-24] & Rosenfeldt E J: "Screenshot der Internet Seite mit eingblendetem Ergebnis der Datumsbestimmung", , 30. September 2011 (2011-09-30), Gefunden im Internet: URL:http://www.tawwa.org/tw11paper/UV%20Ad vanced%20Oxidation%20Treatment%20of%20Emer ging%20Contaminants%20in%20Drinking%20and% 20Reuse%20Water.pdf [gefunden am 2013-05-24]

## Beschreibung

Die vorliegende Erfindung betrifft ein Verfahren zur Bestimmung des Radikalzehrungspotentials eines Wassers, und umfasst die Bestimmung des Abbaus wenigstens einer Referenzsubstanz in dem Wasser infolge Bestrahlung mit UV-Licht bei wenigstens einer Wellenlänge im Bereich von 100 bis 350 nm, und eine Vorrichtung zur Durchführung einer solchen Bestimmung.

### Hintergrund der Erfindung

Zur Steuerung von AOP-Systemen (AOP = Advanced Oxidation Process, englisch für "fortgeschrittenes Oxidationsverfahren") für die oxidative Behandlung, also die oxidative Aufbereitung, Reinigung und/oder Desinfektion von Wasser, werden heutzutage Parameter eingesetzt, welche Auskunft über die Zusammensetzung der Wassermatrix geben und somit nur indirekt die Zehrung der generierten Radikale bestimmen. Momentan werden nur die "klassischen" Parameter wie TOC, Trübung, UV-T und die Messung von spezifischen Parametern wie gelöstes Ozon oder Wasserstoffperoxid zur Steuerung von AOP-Anlagen eingesetzt. Diese Parameter sind aufwendig zu messen und die Geräte hierfür sind meist teuer. Eine Verknüpfung dieser Parameter zu Steuerungszwecken ist komplex und hängt von vielen unterschiedlichen Faktoren ab. Zudem ist die Korrelation zwischen Dosis und Effekt (z.B. Abbau von Spurenstoffen) nicht bekannt oder nur aufwendig zu messen, da stoffspezifische Analysen durchgeführt werden müssen.

Unbekannt ist bisher die Zehrung (der Verbrauch) von OH-Radikalen durch die Wassermatrix und die Messung dieser Zehrung. Momentan werden in einigen Installationen zu viele OH-Radikale produziert (dadurch höherer Verbrauch an Betriebsmitteln), um dem Effekt der "Radikalzehrung" ("Scavenging") vorzubeugen. Die Radikalzehrung wird durch organische und anorganische Substanzen hervorgerufen, die natürlicherweise im Wasser vorhanden sein können, wie zum Beispiel Carbonate.

CN 1869684 A betrifft eine Vorrichtung und ein Verfahren zur Messung des Sauerstoffbedarfs aerober Mirkoorganismen während der Abwasseraufbereitung.

US 2006 240558 A schlägt eine Vorrichtung und ein Verfahren für die Bestimmung des chemischen Sauerstoffbedarfs (COD) einer Wasserprobe vor.

CN 1372141 A offenbart ein Test- und Auswerteverfahren für den organischen Biokörperabbau in Wasser zur Erreichung umfassender Wasserqualitätsziele u. a. unter Heranziehung der Parameter BOD5/COD, CO₂-Bildung und ATP.

JP 9292388 A befasst sich mit einer Methode zur Bestimmung des Abbaus eines Schmierungsdünnfilmes basierend auf einem fluorhaltigen Harz, wobei die Konzentration von Fluorid-Ionen gemessen wird.

EP 0 388 590 A2 betrifft ein Verfahren zur Bestimmung organischer Verbindungen einschließlich aller Abbauprodukte in einer Gas- oder Flüssigphase, wobei das gasförmige oder flüssige Probegut in eine begrenzte Menge Wasser überführt wird, worin die organischen Verbindungen photolytisch zersetzt werden. Die Zersetzungsprodukte werden im Trägergas und/oder in der begrenzten Wassermenge nachgewiesen.

DE 43 16 452 C1 beschreibt ein Verfahren zum Abbau polymerer organischer Schadstoffe in Wasser mittels der AOP-Kombination UV und Wasserstoffperoxid; auf ein Messverfahren wird jedoch nicht hingewiesen.

US 2010/0206787 A1 schlägt ein System zur Behandlung von Flüssigkeiten mittels UV-Strahlung und eines Oxidationsmittels in einem fortgeschrittenen Oxidationsverfahren (advanced oxidation process, AOP) vor. Das System wird durch einen Regelprozess gesteuert, wobei die beschriebene Messtechnik zwei chemische Sensoren benutzt und den Gehalt an Oxidationsmittel, insbesondere Ozon in der flüssigen Phase bestimmt und daraufhin die UV-Dosis steuert.

US 2008/0179178 A1 offenbart einen UV-Reaktor zur Reinigung von Abwasser, ebenfalls basierend auf dem AOP-Verfahren. Es kommt eine UV-Lichtquelle und eine Titandioxidschicht als Katalysator zur Herstellung von Hydroxyl-Radikalen zum Einsatz, wobei es um den Aufbau des dafür benötigten UV-Reaktors für den großtechnischen Einsatz und nicht ein Messverfahren für OH-Radikale geht.

EP 1 008 556 A2 stellt ein Verfahren zur Dekontamination schadstoffbelasteter Abwässer mittels Lichtstrahlung und Ultraschall, also einer kombinierten photo- und sonochemischen Behandlung, vor, wobei zusätzlich auch Oxidationsmittel wie Ozon verwendet werden können; auf ein Messverfahren finden sich jedoch keine Hinweise.

Aus der Internet-Veröffentlichung von Erik J. Rosenfeldt "UV Advanced Oxidation Treatment of Emerging Contaminants in Drinking and Reuse Water" (veröffentlicht 2011 auf http://www.tawwa.org/) ist ein Verfahren zur Bestimmung des Radikalzehrungspotentials eines Wassers bekannt. Dazu wird dem Wasser Methylenblau hinzugefügt, eine vorbestimmte H₂O₂-Konzentration eingestellt, das Gemisch mit UV-Licht bestrahlt und die Abbaukinetik von Methylenblau photometrisch bestimmt. Hierfür wurde ein tragbares Gerät entwickelt.

Ein Verfahren zur Bestimmung des Radikalzehrungspotentials eines Wassers zur effizienten Steuerung des Verbrauchs an Betriebsmitteln in einem AOP-System ist bislang nicht bekannt. Aufgabe der vorliegenden Erfindung ist es daher, den Verbrauch an Betriebsmitteln in ATP-Systemen und Verfahren und Anlagen für die oxidative Behandlung von Wasser zu optimieren oder zu senken. Insbesondere ist es Ziel der vorliegenden Erfindung, ein Verfahren und eine Vorrichtung zur Steuerung eines Prozesses zur oxidativen Behandlung von Wasser anzugeben.

### Beschreibung der Erfindung

Die vorliegende Erfindung stellt ein Verfahren zur Bestimmung des momentanen Radikalzehrungspotentials eines Wassers zur Steuerung der oxidativen Behandlung von Wasser bereit, welches die Bestimmung des Abbaus wenigstens einer Referenzsubstanz in dem Wasser umfasst und wobei folgende Schritte vorgesehen sind:
(1) Zugabe wenigstens einer Referenzsubstanz zu einer Wasserprobe und Vermischen aller vorgenannten Komponenten,
(2) Bestrahlung eines Teils der in Schritt (1) erhaltenen Mischung mit UV-Licht bei wenigstens einer Wellenlänge im Bereich von 100 bis 350 nm in einer Bestrahlungszelle,
(3) Messung der Absorption der in Schritt (2) erhaltenen bestrahlten Mischung mit Licht bei wenigstens einer Wellenlänge im Bereich von 300-700nm, bevorzugt 600 bis 660 nm,
(4) Messung der Absorption eines anderen Teils der in Schritt (1) erhaltenen, unbehandelten Mischung als Vergleichsmischung bei derselben Wellenlänge wie in Schritt (3) als Referenzmessung, und
(5) direkte Bestimmung des Abbaus der Referenzsubstanz als abgebaute Menge der Referenzsubstanz aus der Differenz der in Schritt (3) und (4) erhaltenen Messwerte.

Weiter stellt die Erfindung eine Vorrichtung zur Durchführung des Verfahrens bereit, umfassend eine Steuerungsvorrichtung zur Steuerung der oxidativen Behandlung von Wasser sowie
1) wenigstens einen Vorratsbehälter enthaltend eine Lösung wenigstens einer Referenzsubstanz,
2) wenigstens eine Mischvorrichtung zur Bereitstellung einer Wasserprobe durch Vermischung des Wassers mit der wenigstens einen Referenzsubstanz,
3) ein Zweiweg-UV-Bestrahlungsgerät, worin in einer Kammer oder Zelle (Bestrahlungszelle) die aus der Mischvorrichtung stammende Wasserprobe mit UV-Licht bestrahlt und bevorzugt mit einer anderen Kammer (Vergleichszelle) die Intensität des UV-Lichtes aufgenommen wird, wobei das Zweiweg-UV-Bestrahlungsgerät eine emittierte Wellenlänge von 200 bis 300 nm hat, und 4) ein Zweiwegphotometer, worin in einer Kammer oder Zelle (Messzelle) die Absorption der aus der Bestrahlungszelle stammenden Wasserprobe mit Licht von wenigstens einer Wellenlänge im Bereich von 351 bis 800 nm gemessen wird und in der anderen Kammer (Vergleichsmesszelle) die Absorption der unbehandelten, aus der Mischvorrichtung stammenden Wasserprobe (Vergleichsprobe) bei derselben Wellenlänge wie in der Messzelle gemessen wird.

Der Vorteil der hierin beschriebenen Erfindung ist die direkte Messung des Radikalzehrungsverhaltens eines Wassers durch den radikalischen Abbau einer Referenzsubstanz, beispielsweise von Methylenblau. Durch diesen Parameter kann die Anlage effizienter in Bezug auf die Betriebskosten gesteuert werden, da direkt und unmittelbar die momentane Zehrung der OH-Radikale bestimmt wird und nicht indirekt abgeschätzt werden muss. Die Bestimmung dieses Parameters und dessen Kombination mit einem zweiten (Standard-)Parameter (z.B. UV-T-Messung) und die daraus erfolgende Steuerung eines AOP-Systems bietet erhebliche Vorteile, die bis jetzt nicht bekannt waren, da bisher kein finales Steuerungskonzept existierte.

Das hier beschriebene Online-Überwachungssystem basiert auf dem Effekt, dass eine Referenzsubstanz wie Methylenblau durch Hydroxylradikale abgebaut oder zersetzt werden kann. Die Abbaurate ist durch den organischen und anorganischen Hintergrund des Wassers, zu dem die Referenzsubstanz hinzugefügt wurde, beeinflusst.

Der Abbau der Referenzsubstanz erfolgt in einer Bestrahlungskammer, worin eine definierte UV-Lichtdosis zur Anwendung kommt und die notwendigen Hydroxylradikale gebildet werden. Die UV-Dosis wird registriert und kontrolliert unter Verwendung einer Vergleichskammer, die mit Luft gefüllt ist.

Die Abbaurate der Referenzsubstanz kann mit Hilfe der Messung von sichtbarem Licht, im Falle der Verwendung von Methylenblau als Referenzsubstanz bei einer Wellenlänge von 600 bis 660 nm, einer Vergleichsprobe (nicht mit UV-Licht bestrahlt) und der behandelten Probe bestimmt werden. Das Ergebnis dieser Messung ist das

Radikalzehrungspotential (Radikal-Scavenging-Potential) der Wassermatrix.

Da die Leistung eines oxidativen Behandlungssystems wie fortgeschrittenen Oxidationsverfahren maßgeblich durch den organischen oder anorganischen Hintergrund (Zehrungspotential) des Wassers beeinflusst wird, wäre ein Online-Überwachungssystem, welches das beschriebene Verfahren verwendet, in der Lage, das Behandlungssystem einzustellen und zu kontrollieren, was zu Einsparungen an operativen Kosten führt und sicherstellt, dass alle Behandlungsziele erreicht werden.

Für den Zweck der Steuerung der oxidativen Behandlung von Wasser, worunter insbesondere die oxidative Aufbereitung, Reinigung, und/oder Desinfektion von Wasser, insbesondere durch den Einsatz von verschiedenen Verfahrensschritten wie UV-Strahlung, Wasserstoffperoxid oder Ozon oder einer Kombination hiervon, verstanden werden, und einer Anlage für die vorgenannten Einsatzbereiche, eignet sich die mit dem erfindungsgemäßen Verfahren ermittelte Abbaurate als Steuer- oder Messwert: Von der ermittelten Radikalzehrungsrate hängt die Dosierung der an der Radikalbildung beteiligten Verfahrensschritte (Radikalbildner), also vor allem der Bestrahlung des Wassers mit UV-Strahlung, der dem Wasser zugemischten Menge Wasserstoffperoxid und/oder der dem Wasser zugemischten Menge Ozon, ab. Je höher die ermittelte Zehrungsrate ist, desto höher wird wenigstens ein Radikalbildner dosiert, je niedriger die ermittelte Abbaurate ist, desto niedriger wird wenigstens ein Radikalbildner dosiert. Durch diese Art der Steuerung kann ein optimaler, möglichst sparsamer Einsatz oder Verbrauch der Radikalbildner bzw. Betriebsmittel gewährleistet werden.

Besonders wirksam in der oxidativen Behandlung von Wasser und daher erfindungsgemäß bevorzugt sind Kombinationen der eingesetzten Radikalbildner, also jeweils UV-Strahlung und Wasserstoffperoxid, UV-Strahlung und Ozon, Wasserstoffperoxid und Ozon und UV-Strahlung, Wasserstoffperoxid und Ozon.

Das im Schritt (2) beschriebene und zur Bestrahlung des Wassers verwendete UV-Licht hat eine Wellenlänge von 100 bis 350 nm, bevorzugt von 150 bis 300 nm, besonders bevorzugt 254 nm. Die Bestrahlung der Wasserprobe, die z.B. Wasserstoffperoxid enthalten kann, mit UV-Licht dient zur Herstellung der Hydroxylradikale. Diese Bestrahlung findet in einer definierten Zeitspanne statt. Beispielsweise kann eine vorgewählte Zeitspanne zwischen 1 Sekunde und 1 Stunde vorgesehen sein.

In dem erfindungsgemäßen Verfahren kann die Intensität des in Schritt (2) verwendeten UV-Lichtes durch eine gleichzeitig erfolgende Vergleichsmessung mit einer Vergleichszelle, die mit einem Gas, bevorzugt mit Luft gefüllt ist, bestimmt und registriert werden.

Die Wahl der Wellenlänge des Lichtes, das zur Messung der Absorption der in Schritt (3) erhaltenen bestrahlten Mischung zum Einsatz kommt, hängt von der verwendeten Referenzsubstanz, insbesondere von deren Absorptionsmaxima ab, d.h. bevorzugt kommt Licht der Wellenlänge(n) zum Einsatz, bei denen die Absorptionsmaxima der Referenzsubstanz liegen. Erfindungsgemäß kann Licht bei wenigstens einer Wellenlänge im Bereich von 300-700nm, bevorzugt 600 bis 660 nm, verwendet werden. Die Messung der Absorption erfolgt bevorzugt photometrisch.

Die Referenzsubstanz(en) ist bevorzugt ein Stoff, der im sichtbaren Licht im Wellenlängebereich von 300 bis 700 nm absorbiert. Besonders bevorzugt als Referenzsubstanz ist der Farbstoff Methylenblau.

Durch die Bestrahlung der Referenzprobe und der behandelten Probe aus Schritt (2) wird die Färbung des Wassers, die durch die Referenzsubstanz hervorgerufen wird, gemessen. Die Bestrahlung erfolgt vorzugsweise
(a) in Schritt (3) über einen vorwählbaren oder vorgewählten Zeitraum, z.B. zwischen 1 Sekunde und 1 Stunde, und
(b) in Schritt (4) die Messung zeitlich parallel zur Messung in Schritt (3) durchzuführen, wie in (a) beschrieben.

Die Bestimmung des Abbaus der Referenzsubstanz umfasst insbesondere die Bestimmung der Abbaurate dieser Referenzsubstanz. Diese Abbaurate kann insbesondere durch eine Auftragung in Schritt (5) gewonnenen Differenzwerte bestimmt werden.

In dem erfindungsgemäßen Verfahren erfolgen die Schritte (1) bis (5) bevorzugt zeitlich nacheinander entsprechend der Nummerierung, d.h. Schritt (2) folgt auf Schritt (1), Schritt (3) folgt auf Schritt (2) usw. Bevorzugt erfolgen die Schritte (3) und (4) zeitlich parallel oder zeitlich im Wesentlichen parallel, um so die Intensitäten bei den einzelnen Absorptionswellenlängen unabhängig von den apparativen Gegebenheiten wie der Eigenabsorption der Messzelle wiedergeben zu können.

Das erfindungsgemäße Verfahren eignet sich insbesondere zur Steuerung von Systemen zur oxidativen Behandlung von Wasser, insbesondere der oxidativen Aufbereitung, Reinigung und/oder Desinfektion von Wasser. Die Systeme können aus einer Kombination der folgenden Komponenten bestehen, wobei hier zusätzlich der zu regelnde Parameter beschrieben wird:
(a) die Bestrahlung mit UV-Licht im Bereich von 100 bis 350 nm und die Steuerung dieser Bestrahlung,
(b) die Zugabe von Wasserstoffperoxid und die Steuerung der Zugabe von Wasserstoffperoxid,
(c) die Zugabe von Ozon und die Steuerung der Dosierung von Ozon.

Das Wasser, das die aktuelle Konzentration an Wasserstoffperoxid enthält, wird über einen Bypass zur Vorrichtung geführt. Sollte das Wasser prozessbedingt kein Wasserstoffperoxid enthalten, muss dieses vor der Vorrichtung durch eine Mischeinheit dem Wasser zugeführt werden.

Die Vorrichtung zur Durchführung des erfindungsgemäßen Verfahrens umfasst eine Steuerungsvorrichtung zur Steuerung der oxidativen Behandlung von Wasser sowie
1) wenigstens einen Vorratsbehälter enthaltend eine bevorzugt wässerige Lösung wenigstens einer erfindungsgemäß verwendeten Referenzsubstanz,
2) wenigstens eine Mischvorrichtung zur bevorzugt homogenen Vermischung des aus dem vorstehend beschriebenen Wassers mit wenigstens einer Referenzsubstanz (1),
3) ein Zweiweg-UV-Bestrahlungsgerät, worin in einer Kammer oder Zelle (Bestrahlungszelle) die aus der unter (2) definierten Mischvorrichtung stammende Wasserprobe mit UV-Licht bestrahlt und bevorzugt mit einer anderen Kammer (Vergleichszelle) die Intensität des UV-Lichtes aufgenommen wird, wobei das Zweiweg-UV-Bestrahlungsgerät eine Wellenlänge von 200 bis 300 nm hat, und
4) ein Zweiwegphotometer, worin in einer Kammer oder Zelle (Messzelle) die Absorption der aus der Bestrahlungszelle in (3) stammenden Wasserprobe mit Licht von wenigstens einer Wellenlänge im Bereich von 351 bis 800 nm gemessen wird und in der anderen Kammer (Vergleichsmesszelle) die Absorption der unbehandelten, aus der unter (2) definierten Mischvorrichtung stammenden Wasserprobe (Vergleichsprobe) bei derselben Wellenlänge wie in der Messzelle gemessen wird.

Die Vorrichtung kann auch ein Behandlungssystem bzw. AOP-System für die oxidative Behandlung, insbesondere die oxidative Aufbereitung, Reinigung, und/oder Desinfektion von Wasser, umfassen und dieses Behandlungssystem steuern.

### Beschreibung der Figuren

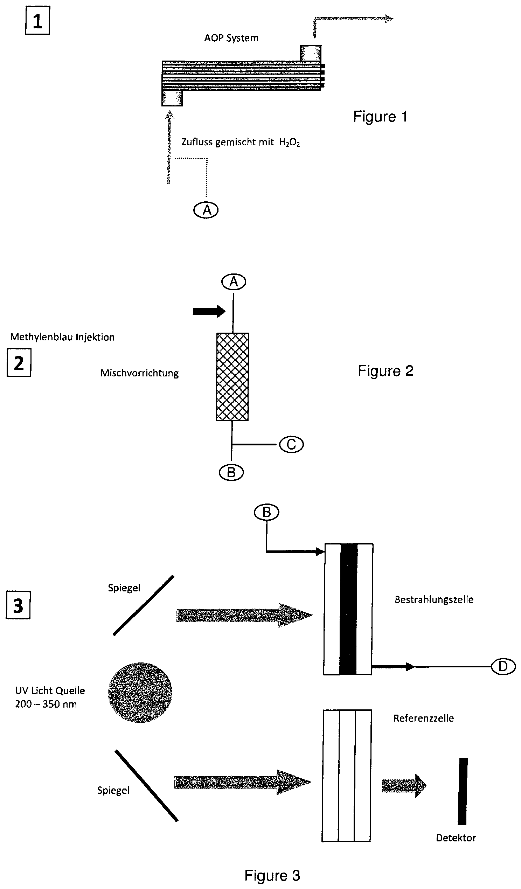

Anhand der Zeichnung mit den Figuren 1 bis 4 werden das Verfahren nach einer Ausführungsform der Erfindung und die apparativen Komponenten hierfür beschrieben.
- Figur 1: zeigt das AOP-System (AOP = Advanced Oxidation Process). Das Wasser wird mit Wasserstoffperoxid vermischt und in diesem Fall an einer UV-Bestrahlung (UV-Reaktor) vorbeigeleitet. Ebenso kann die UV-Bestrahlung durch ein Ozonsystem ersetzt werden. Ein Teil des Wassers wird vor dem Zulauf des Behandlungssystems in das OnlineÜberwachungssystem umgeleitet und an die Baugruppe in Figur 2 übergeben.

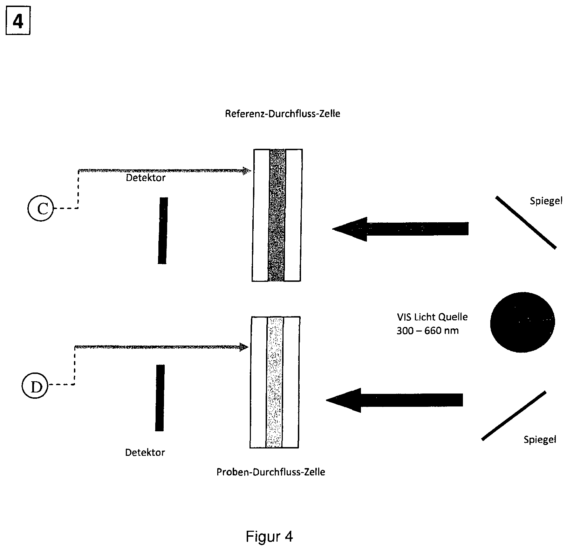

- Figur 2: zeigt die Methylenblau-Zugabe. Eine Methylenblau-Vorratslösung wird dem Wasser hinzugefügt und auf eine Art und Weise vermischt, dass das gesamte Methylenblau in der Wasserprobe homogen verteilt wird. Ein Teil dieser Mischung geht zu der Bestrahlung mit UV-Strahlung in der Baugruppe in Figur 3. Ein anderer Teil dieser Mischung wird als Referenzprobe zu Figur 4 weitergeleitet.
- Figur 3: zeigt ein Zweiweg-UV-Bestrahlungsgerät, worin in einer Kammer oder Zelle die Wasserprobe aus Figur 2 mit UV-Licht bestrahlt und in der anderen Kammer (Vergleichszelle) die Intensität des UV-Lichtes aufgenommen wird. Nach der Bestrahlung wird die Wasserprobe zu Schritt 4 (Figur 4) weitergeleitet.
- Figur 4: zeigt ein Zweiwegphotometer, worin in einer Kammer oder Zelle die Absorption der UV-bestrahlten Wasserprobe aus Figur 3 bei Wellenlängen von 600 bis 660 nm gemessen wird und in der anderen Kammer die Absorption der unbehandelten Probe aus Methylenblau gemischt mit Wasser (Vergleichsprobe) aus Figur 2 bei denselben Wellenlängen gemessen wird.

Dieses Verfahren bestimmt direkt den Abbau des Methylenblaus durch die Differenz in der Absorption. Nach der Messung kann die Wasserprobe verworfen oder zum Wasserbehandlungssystem zurückbefördert werden.

## Patentansprüche

1. Verfahren zur Bestimmung des momentanen Radikalzehrungspotentials eines Wassers zur Steuerung der oxidativen Behandlung von Wasser, umfassend die Bestimmung des Abbaus wenigstens einer Referenzsubstanz in dem Wasser wobei folgende Schritte vorgesehen sind:
(1) Zugabe wenigstens einer Referenzsubstanz zu einer Wasserprobe und Vermischen aller vorgenannten Komponenten,
(2) Bestrahlung eines Teils der in Schritt (1) erhaltenen Mischung mit UV-Licht
bei wenigstens einer Wellenlänge im Bereich von 100 bis 350 nm in einer Bestrahlungszelle,
(3) Messung der Absorption der in Schritt (2) erhaltenen bestrahlten Mischung mit Licht bei wenigstens einer Wellenlänge im Bereich von 300-700nm, bevorzugt 600 bis 660 nm,
(4) Messung der Absorption eines anderen Teils der in Schritt (1) erhaltenen, unbehandelten Mischung als Vergleichsmischung bei derselben Wellenlänge wie in Schritt (3) als Referenzmessung, und
(5) direkte Bestimmung des Abbaus der Referenzsubstanz als abgebaute Menge der Referenzsubstanz aus der Differenz der in Schritt (3) und (4) erhaltenen Messwerte.

2. Verfahren nach Anspruch 1, wobei die Radikale Hydroxylradikale sind.

3. Verfahren nach Anspruch 1 oder 2, wobei zur Bildung der Radikale Wasserstoffperoxid eingesetzt wird.

4. Verfahren nach einem der Ansprüche 1 bis 3, wobei die Referenzsubstanz ein Stoff ist, der im sichtbaren Licht im Wellenlängebereich von 300 bis 700 nm absorbiert, bevorzugt Methylenblau.

5. Verfahren nach einem der Ansprüche 1 bis 4, zusätzlich umfassend die gleichzeitige Aufnahme der Intensität des UV-Lichtes in Schritt (2) durch eine Vergleichsmessung mit einer Vergleichszelle.

6. Verfahren nach einem der Ansprüche 1 bis 5, wobei
(a) in Schritt (2) die Bestrahlung über einen vorwählbaren Zeitraum von 1 Sekunde bis 1 Stunde erfolgt,
(b) in Schritt (3) die Messung über einen vorwählbaren Zeitraum von 1 Sekunde bis 1 Stunde erfolgt, und/oder
(c) in Schritt (4) die Messung zeitlich parallel zur Messung in Schritt (3) erfolgt.

7. Verfahren nach einem der Ansprüche 1 bis 7, wobei die Bestimmung des Abbaus wenigstens einer Referenzsubstanz die Bestimmung der Abbaurate dieser Referenzsubstanz umfasst.

8. Verfahren nach einem der Ansprüche 2 bis 7, wobei durch eine zeitliche Auftragung von wenigstens zwei der in Schritt (5) gewonnenen Differenzwerte die Abbaurate wenigstens einer Referenzsubstanz bestimmt wird.

9. Verfahren nach einem der Ansprüche 1 bis 8, wobei die Schritte (1) bis (5) zeitlich nacheinander von Schritt (1) bis Schritt (5) ausgeführt werden, wobei bevorzugt die Schritte (3) und (4) zeitlich parallel oder zeitlich im wesentlichen parallel ausgeführt werden.

10. Verfahren nach einem der vorhergehenden Ansprüche, wobei die oxidative Behandlung
(a) die Bestrahlung mit UV-Licht im Bereich von 100 bis 350 nm und die Steuerung dieser Bestrahlung, und/oder
(b) die Zugabe von Wasserstoffperoxid und die Steuerung der Zugabe von Wasserstoffperoxid, und/oder
(c) die Zugabe von Ozon und die Steuerung der Dosierung von Ozon umfasst.

11. Vorrichtung angepasst zur Durchführung eines Verfahrens wie in den Ansprüchen 1 bis 10 definiert und umfassend eine Steuerungsvorrichtung zur Steuerung der oxidativen Behandlung von Wasser sowie
1) wenigstens einen Vorratsbehälter enthaltend eine Lösung wenigstens einer Referenzsubstanz,
2) wenigstens eine Mischvorrichtung zur Bereitstellung einer Wasserprobe durch Vermischung des Wassers mit der wenigstens einen Referenzsubstanz,
3) ein Zweiweg-UV-Bestrahlungsgerät, worin in einer Kammer oder Zelle (Bestrahlungszelle) die aus der Mischvorrichtung stammende Wasserprobe mit UV-Licht bestrahlt und bevorzugt mit einer anderen Kammer (Vergleichszelle) die Intensität des UV-Lichtes aufgenommen wird, wobei das Zweiweg-UV-Bestrahlungsgerät eine emittierte Wellenlänge von 200 bis 300 nm hat, und
4) ein Zweiwegphotometer, worin in einer Kammer oder Zelle (Messzelle) die Absorption der aus der Bestrahlungszelle stammenden Wasserprobe mit Licht von wenigstens einer Wellenlänge im Bereich von 351 bis 800 nm gemessen wird und in der anderen Kammer (Vergleichsmesszelle) die Absorption der unbehandelten, aus der Mischvorrichtung stammenden Wasserprobe (Vergleichsprobe) bei derselben Wellenlänge wie in der Messzelle gemessen wird.

## Claims

1. Procedure for determining the present radical scavenging potential of water to regulate the oxidative treatment of water, which includes determining the degradation of at least one reference substance in the water wherein the following steps are provided:
(1) adding at least one reference substance to a water sample and mixing all the aforementioned components,
(2) irradiation of part of the mixture obtained in step (1) with a UV light
with at least one wavelength in the range of 100 to 350 nm in an irradiation cell,
(3) measuring the absorption of the mixture obtained in step (2) irradiated with light with at least one wavelength in the range of 300 - 700 nm, preferably 600 to 660 nm,
(4) measuring the absorption of another part of the untreated mixture obtained in step (1) as a comparison mixture at the same wavelength as in step (3) as a reference measurement, and
(5) direct determination of the degradation of the reference substance as a degraded quantity of the reference substance from the difference between the measured values obtained in steps (3) and (4).

2. Procedure according to Claim 1, wherein the radicals are hydroxyl radicals.

3. Procedure according to Claim 1 or 2, wherein hydrogen peroxide is used to form the radicals.

4. Procedure according to any one of Claims 1 to 3, wherein the reference substance is a material which absorbs visible light in the wavelength of 300 to 700 nm, preferably methylene blue.

5. Procedure according to any one of Claims 1 to 4, additionally comprising the simultaneous recording of the intensity of the UV light in step (2) by a comparison measurement with a comparison cell.

6. Procedure according to any one of Claims 1 to 5, wherein
(a) in step (2) the irradiation takes place over a pre-selectable time period of 1 second to 1 hour,
(b) in step (3) the measurement takes place over a pre-selectable time period of 1 second to 1 hour, and/or
(c) in step (4) the measurement takes place at the same time as the measurement in step (3).

7. Procedure according to any one of Claims 1 to 7, wherein the determination of the degradation of at least one reference substance comprises the determination of the degradation rate of this reference substance.

8. Procedure according to any one of Claims 2 to 7, wherein the degradation rate of at least one reference substance is determined by a consecutive application of at least two difference values acquired in step (5).

9. Procedure according to any one of Claims 1 to 8, wherein steps (1) to (5) are performed consecutively one after the other from step (1) to (5), wherein steps (3) and (4) are preferably performed at the same time or essentially at the same time.

10. Procedure according to any one of the previous claims, wherein the oxidative treatment comprises
(a) the irradiation with UV light in the range of100 to 350 nm and the regulation of this irradiation, and/or
(b) the addition of hydrogen peroxide and the regulation of the addition of hydrogen peroxide, and/or
(c) the addition of ozone and the regulation of this dosage of ozone.

11. Device adapted to implement the procedure defined in Claims 1 to 10 comprising a regulation device for regulating the oxidative treatment of water as well as
1) at least one reservoir containing a solution of at least one reference substance,
2) at least one mixing device for preparing a water sample by means of mixing the water with at least one reference substance,
3) a two-way UV irradiation device wherein the water sample from the mixing device is irradiated with UV light in a chamber or cell (irradiation cell), and the intensity of the UV light is preferably incorporated with another chamber (comparison cell), wherein the two-way UV irradiation device has an emitted wavelength of 200 to 300 nm, and
4) a two-way photometer wherein the absorption of the water sample from the irradiation cell is measured with light of at least one wavelength in the range of 351 to 800 nm in a chamber or cell (measuring cell), and in the other chamber (comparison measuring cell) the absorption of the untreated water sample from the mixing device (comparison sample) is measured at the same wavelength used in the measuring cell.

## Revendications

1. Procédé pour déterminer le potentiel de consommation de radicaux d'une eau dans le but de réguler un processus de traitement oxydatif de l'eau, qui comprend la détermination de la décomposition d'au moins une substance de référence dans l'eau, auquel cas les étapes suivantes sont prévues:
(1) introduction d'au moins une substance de référence dans un échantillon d'eau et le mélange de l'ensemble des composants précités,
(2) irradiation du mélange obtenu dans l'étape (1) avec de la lumière ultraviolette (UV) par au moins une longueur d'onde dans une plage allant de 100 à 350 nm dans une cellule d'irradiation,
(3) mesure de l'absorption du mélange obtenu dans l'étape (2) irradié avec de la lumière par au moins une longueur d'onde dans une plage de 300-700 nm, de préférence de 600 à 660 nm,
(4) mesure de l'absorption du mélange non traité obtenu dans l'étape (1) en tant que mélange comparatif par la même longueur d'onde comme dans l'étape (3) en tant que mesure de référence, et
(5) détermination directe de la décomposition de la substance de référence en tant que quantité décomposée de la substance de référence à partir de la différence des valeurs de mesure obtenues dans l'étape (3) et (4).

2. Procédé selon la revendication 1, auquel cas les radicaux sont des radicaux hydroxyles.

3. Procédé selon la revendication 1 ou 2, auquel cas l'on utilise du peroxyde d'hydrogène pour la formation des radicaux.

4. Procédé selon une des revendications de 1 à 3, auquel cas la substance de référence est une substance qui est absorbée en lumière visible dans une plage de longueur d'onde de 300 à 700 nm, de préférence du bleu de méthylène.

5. Procédé selon une des revendications de 1 à 4, comprenant en outre l'absorption simultanée de l'intensité de la lumière ultraviolette (UV) dans l'étape (2) par l'intermédiaire d'une mesure de comparaison avec une cellule témoin.

6. Procédé selon une des revendications de 1 à 5, auquel cas
(a) dans l'étape (2) l'irradiation a lieu sur un laps de temps présélectionnable allant de 1 seconde jusqu'à 1 heure,
(b) dans l'étape (3) la mesure a lieu sur un laps de temps présélectionnable allant de 1 seconde jusqu'à 1 heure, et/ou
(c) dans l'étape (4) la mesure a lieu simultanément en parallèle par rapport à la mesure dans l'étape (3).

7. Procédé selon une des revendications de 1 à 7, auquel cas la détermination de la décomposition d'au moins une substance de référence comporte la détermination de la vitesse de décomposition de cette substance de référence.

8. Procédé selon une des revendications de 2 à 7, auquel cas l'on détermine par une application temporelle d'au moins deux des écarts obtenus dans l'étape (5) la vitesse de décomposition d'au moins une substance de référence.

9. Procédé selon une des revendications de 1 à 8, auquel cas l'on exécute les étapes de (1) à (5) successivement dans le temps à partir de l'étape (1) jusqu'à l'étape (5), auquel cas l'on exécute de préférence les étapes (3) et (4) parallèlement dans le temps ou essentiellement parallèlement dans le temps.

10. Procédé selon une des revendications précédentes, auquel cas le traitement oxydatif comporte
a) l'irradiation avec de la lumière ultraviolette (UV) dans une plage allant de 100 à 350 nm et la régulation de cette irradiation, et/ou
b) l'introduction de peroxyde d'hydrogène et la régulation de l'introduction de du peroxyde d'hydrogène, et/ou
c) l'introduction d'ozone et la régulation du dosage d'ozone.

11. Dispositif adapté pour réaliser un tel procédé tel que définit dans les revendications de 1 à 10 et comprenant un dispositif de régulation dans le but de réguler un processus de traitement oxydatif de l'eau ainsi que
1) au moins un réservoir contenant une solution à base d'au moins une substance de référence,
2) au moins un dispositif mélangeur pour la préparation d'un échantillon d'eau par l'intermédiaire d'un mélange d'eau avec au moins une substance de référence,
3) un appareil d'irradiation aux rayons ultraviolets (UV) à deux directions, dans lequel l'échantillon d'eau provenant du dispositif mélangeur est irradié avec une lumière ultraviolette (UV) dans une chambre ou cellule (cellule d'irradiation) et de préférence l'intensité de la lumière ultraviolette (UV) est absorbée avec une autre chambre (cellule témoin), auquel cas l'appareil d'irradiation aux rayons ultraviolets (UV) à deux directions présente une longueur d'onde émise de 200 à 300 nm, et
4) un photomètre à deux directions, dans lequel dans une chambre ou cellule (cellule de mesure) l'absorption de l'échantillon d'eau provenant de la cellule d'irradiation est mesurée avec de la lumière d'au moins une longueur d'onde dans une plage allant de 351 à 800 nm et dans l'autre chambre (cellule de mesure témoin) l'absorption de l'échantillon d'eau non traité (échantillon témoin) provenant du dispositif mélangeur est mesurée par la même longueur d'onde comme dans la cellule de mesure.
